Europäisches Patentamt

European Patent Office

Office européen des brevets

(19)

(11) Veröffentlichungsnummer: **0 160 274**
**B1**

(12) **EUROPÄISCHE PATENTSCHRIFT**

(45) Veröffentlichungstag der Patentschrift: **25.07.90**

(21) Anmeldenummer: **85105075.7**

(22) Anmeldetag: **26.04.85**

(51) Int. Cl.⁵: **A 61 F 5/02**

(54) **Elektrische Spannvorrichtung für orthopädische Justiervorrichtungen.**

(30) Priorität: **30.04.84 CH 2106/84**

(43) Veröffentlichungstag der Anmeldung:
**06.11.85 Patentblatt 85/45**

(45) Bekanntmachung des Hinweises auf die
Patenterteilung:
**25.07.90 Patentblatt 90/30**

(84) Benannte Vertragsstaaten:
**AT DE FR GB IT NL SE**

� Entgegenhaltungen:
**DE-A-2 845 647**
**GB-A-2 098 490**
**US-A-3 889 664**

(73) Patentinhaber: **Wiederkehr, Hans, Dr.**
**Ifangstrasse 107**
**CH-8153 Rümlang (CH)**

(72) Erfinder: **Wiederkehr, Hans, Dr.**
**Ifangstrasse 107**
**CH-8153 Rümlang (CH)**

(74) Vertreter: **EGLI-EUROPEAN PATENT**
**ATTORNEYS**
**Horneggstrasse 4**
**CH-8008 Zürich (CH)**

## Beschreibung

Die Erfindung betrifft eine elektrische Spannvorrichtung nach dem Oberbegriff des Patentanspruchs 1 (DE—A—2845647).

Für Träger einer orthopädischen Vorrichtung, z.B. eines Stützkorsetts zur Entlastung der Wirbelsäule bei Bandscheibenschäden können auftretende Schmerzen zwar durch Schmerzmittel gelindert werden, jedoch stellt dies wegen den damit verbundenen Nachteilen eine recht wenig befriedigende Lösung der. Aus diesem Grunde wird nach anderen Lösungen gesucht wobei auch Mittel zur Ausübung von Kräften an den Justiervorrichtungen (DE—A—2845647), z.B. zur Aenderung der Spannwirkung eines Stützkorsetts, in Erwägung gezogen wurden, doch haben sich bekannte Mittel hierzu als wenig geeignet erwiesen.

Hier setzt die Erfindung ein, der die Aufgabe zugrundeliegt, eine Spannvorrichtung der eingangs beschriebenen Art so auszugestilten, dass einerseits ein sehr geringes Bauvolumen eingehalten und andererseits eine sehr genaue Längeneinstellung der Spannvorrichtung erreicht werden kann. Diese Aufgabe wird gemäss der Erfindung durch die kennzeichnenden Merkmale des Patentanspruchs 1 gelöst.

Die Erfindung ist in der Zeichnung im mehreren Ausführungsbeispielen dargestellt und nachfolgend beschrieben. Es zeigen:

Fig. 1 einen Längsschnitt einer Spannvorrichtung,

Fig. 2 den Bereich der Axiallagerung des Spindeltriebs der Spannvorrichtung nach Fig. 1,

Fig. 3 einen Längsschnitt einer zweiten Spannvorrichtung mit Hubeinstellung,

Fig. 4 einen Längsschnitt einer Spannvorrichtung nach Fig. 1 mit gefederter Führungsbüchse,

Fig. 5 einen Längsschnitt einer dritten Spannvorrichtung mit Schrittmotor und Positionsgeber,

Fig. 6 einen Längsschnitt einer vierten Spannvorrichtung mit Schrittmotor und untersetzter Doppelspindel,

Fig. 7 einen Längsschnitt einer fünften Spannvorrichtung mit Schrittmotor und Kugelgewindespindel und

Fig. 8 einen Längsschnitt einer sechsten Spannvorrichtung mit auswechselbarer Gewindespindel.

Die Erfindung geht von der Ueberlegung aus, dass Justiervorrichtungen für orthopädische Zweck nur dann dem Patienten volle Befriedigung bieten, wenn eine an der Vorrichtung einmal gewählte Stellung über lange Zeit, d.h mehrere Stunden, unverändert erhalten bleibt, wobei aber auch eine äusserst feine und exakte Einstellung der Vorrichtung erforderlich ist. Dies kann durch die nachstehend beschriebenen Ausführungsbeispiele der Erfindung erreicht werden.

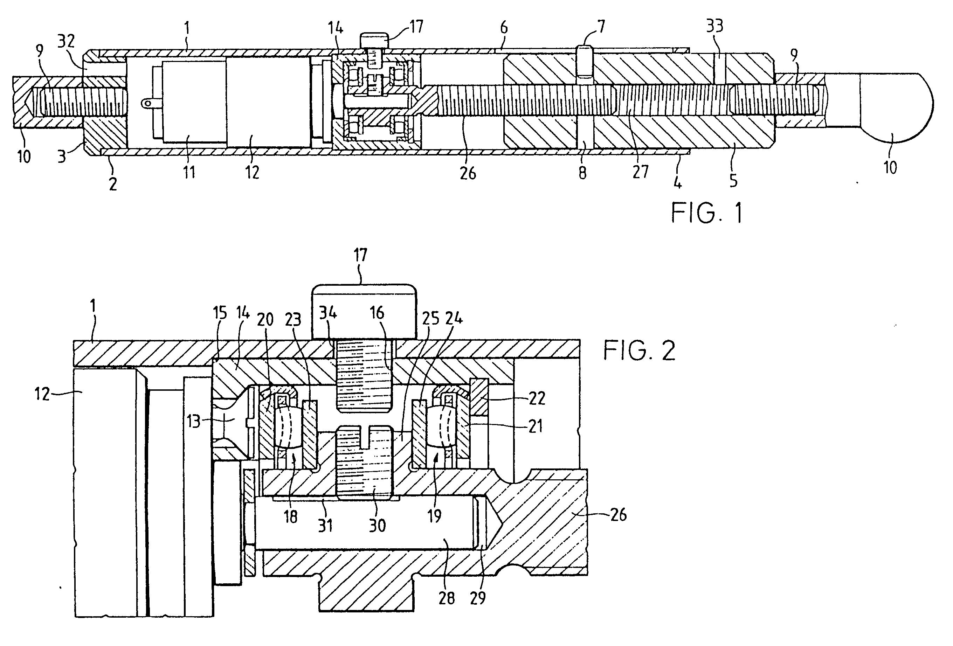

Die Spannvorrichtung nach Fig. 1 weist ein Hohlprofil 1 auf, das an seinem einen Ende durch einen Boden 3 abgeschlossen ist. Der Boden 3 kann jedoch auch weggelassen werden, wodurch die Ausbaubarkeit der im Innern des Hohlprofils 1 angeordneten Teile erleichtert wird. Das Hohlprofil 1 ist an seinem anderen Ende 4 offen und bildet die Führung für einen Hubkörper 5, der in dem Hohlprofil 1 längsverschiebbar geführt ist. Hierzu ist ein Längsschlitz 6 vorgesehen, in den ein Bolzen 7, z.B. ein Querspannstift, ragt, der in einer Radialbohrung 8 des Hubkörpers 5 gelagert ist. In den Boden 3 ist ein Gewindebolzen 9 eingeschraubt, auf dessen vorstehendes Ende ein zur teilweise dargestellter Gelenkbolzen 10 aufgeschraubt ist. Auf der gegenüberliegenden Seite des Hohlprofils 7 ist auf der einen Stirnseite des Hubkörpers 5 ein gleicher Gewindebolzen 9 mit einem Gelenkbolzen 10 in dem Hubkörper 5 eingeschraubt. Der Gelenkbolzen 10 ist ein Teil eines Winkelgelenkes, dessen Gegenstück in der Justiervorrichtung befestigt ist und in einem Gelenkkopf endet, auf dem eine am Ende des Gelenkbolzens 10 angeordnete Kugelpfanne abgestützt ist. Für den gegenüberliegenden andern Gelenkbolzen 10 ist ein weiterer Gelenkkopf abgestützt, z.B. ebenfalls in der Justiervorrichtung.

In dem Mantelrohr 1 ist ein Niederspannungs-Elektromotor 11 mit einem Reduktionsgetriebe 12 untergebracht, welch letzteres mittels Schrauben, z.B. Senkschrauben 13 (Fig. 2), an einer Lagerbüchse 14 abgestützt ist. Die Lagerbüchse 14 ist in das Hohlprofil 1 eingesetzt und seine Lage ist durch eine Schulter 15 im Hohlprofil 1 definiert. In der Lagerbüchse 14 ist eine Gewindebohrung 16 angeordnet, in die eine von aussen durch eine Bohrung 34 in dem Hohlprofil 1 ragende Schraube 17 eingeschraubt ist und die Lagerbüchse 14 in ihrer Lage fixiert.

Im Innern der Lagerbüchse 14 sind zwei Axialwählzlager 18, 19, z.B. Axial-Nadellager, angeordnet, deren Lagerringe 20, 21 am Boden der Lagerbüchse 14 bzw. an einem in der Lagerbüchse 14 gelagerten Sprengring 22 abgestützt sind, während die entsprechenden Gegenringe 23, 24 an einem Bunde 25 anliegen, der einen Teil einer Spindel 26 bildet. Die Spindel 26 ist auf der einen Seite in den Hubkörper 5 eingeschraubt, der mit seiner Gewindebohrung 27 die Mutter eines Spindeltriebes bildet, dessen Spindel die Spindel 26 ist. Das mit dem Elektromotor 11 verbundene Reduktionsgetriebe 12 weist eine Getriebewelle 28 auf. Auf der Getriebewelle 28 wird das Ende der Spindel 26 mit einer Bohrung 29 aufgeschoben. Im Bund 25 ist eine Sicherung in Form eines Gewindezapfens 30 eingeschraubt, der mit seiner Stirnfläche in eine in der Getriebewelle 28 eingearbeitete Nut 31 ragt und die Mitnahme zwischen der Getriebewelle 28 und der Spindel 26 sicherstellt. Wie aus Fig. 2 ersichtlich ist, kann nach Entfernen der Schraube 17 der Gewindezapfen 30 gelöst oder gespannt werden, wodurch die Verbindung zwischen der Getriebewelle 28 und der Spindel 26 gelöst oder gespannt werden kann.

Im Boden 3 ist eine Bohrung 32 vorgesehen, durch die ein elektrisches Kabel gezogen werden kann, mit dem eine Verbindung zwischen dem Elektromotor 11 und einer Kraftquelle, z.B. einer Batterie, hergestellt werden kann.

Eine weitere Bohrung 33 im Hubkörper 5 dient der Entlüftung der Gewindebohrung 27 des Hubkörpers 5.

Wird zur Erreichung einer besseren Zugänglichkeit zu den im Inneren des Hohlprofils 1 angeordneten Teilen der Boden 3 weggelassen, kann der Gelenkbolzen 10 an zwei Zapfen auf gegenüberliegenden Seiten des Hohlprofils 1 schwenkbar gelagert werden. Im übrigen können anstelle der Gelenkrohre auch andere Verbindungsmittel, z.B. Laschen oder Gewindezapfen, vorgesehen werden, die mit entsprechenden Verbindungsteilen an der Justiervorrichtung oder an Stützvorrichtungen verbunden werden. Durch die besondere Anordnung der Teile kann die beschriebene Spannvorrichtung leicht montiert und demontiert werden. Zunächst muss der Bolzen 7 in den Hubkörper 5 eingetrieben werden, worauf der Hubkörper 5 herausgeschraubt werden kann. Dann kann durch Entfernen der Schraube 17 der Gewindezapfen 30 gelöst werden, worauf die Lagerbüchse 14, das Reduktionsgetriebe 12 und der Elektromotor 11 zusammen aus dem Hohlprofil 1 herausgenommen werden können. Die weitere Zerlegung erfolgt durch Entfernen des Sprengrings 22, wodurch die Spindel 26 von der Getriebewelle 28 gelöst werden kann. Die Montage erfolgt in derselben, jedoch umgekehrter Riehenfolge.

Trotz des verhältnismässig engen Mantelrohrs 2 gelingt es, eine platzsparende Spannvorrichtung zu schaffen. Hierbei lassen sich auch sehr hohe Drehzahlreduktionen, z.B. etwa 150:1 erreichen. Wegen dieser grossen Einstellgenauigkeit gelingt es, die Justiervorrichtungen so einzustellen, dass sie dem Patienten Linderung bringen.

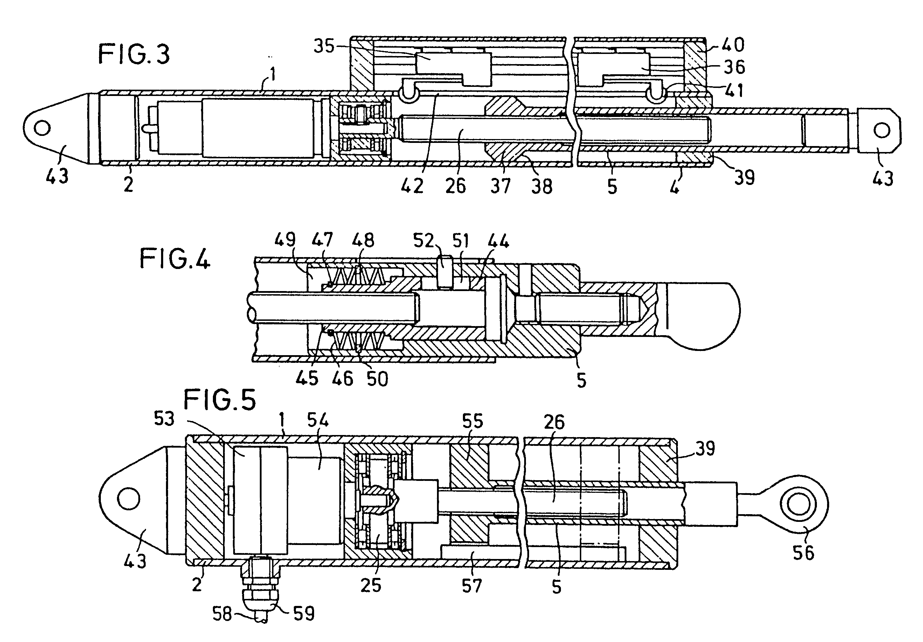

In Fig. 3—7 sind Ausführungsformen von Spannvorrichtungen dargestellt, die wie diejenige nach Fig. 1 bei orthopädischen Justiervorrichtungen eingesetzt werden können, aber noch zusätzliche Elemente aufweisen. In den Figuren 3—7 bedeuten Bezugszahlen, die mit solchen in Fig. 1 übereinstimmen, gleiche Teile, die nur zur Erläuterung der Gesamtfunktion erwähnt werden.

Die Spannvorrichtung nach Fig. 3 unterscheidet sich von derjenigen nach Fig. 1 durch den Anbau von verschiebbaren Endschaltern 35, 36, mit denen der Hub des Spindeltriebs eingestellt werden kann. Zu diesem Zweck ist der Hubkörper 5 im Bereich des die Spindelmutter bildenden Teils als ein Kopf 37 mit Anschrägungen 38 ausgebildet. An den Anschrägungen 38 laufen die Tastrollen der Endschalter 35, 36 auf. Da in diesem Fall der Hubkörper 5 einen kleineren Durchmesser aufweist als der Innendurchmesser des Hohlprofils und deshalb nur im Bereich des Kopfes 37 darin definiert ist, ist am andern Ende 4 ein Führungsring 39 für die Führung des Hubkörperteils mit kleinerem Durchmesser angeordnet.

Die Endschalter 35, 36 sind in einem Gehäuse 40 eingebaut, das auf dem Aussendurchmesser des Hohlprofils 1 befestigt, z.B. aufgeschraubt, ist. Zum Einstellen der Endschalter 35, 36 wird das Gehäuse oder ein Teil desselben entfernt. Die Tastrollen 41 ragen hierbei durch einen Schlitz 42 ins Innere des Hohlprofils 1. Zudem wird der Hubkörper 5 derart geführt, dass während der Hubverstellung keine Rotation des Hubkörpers eintreten kann. Anstelle der Gelenkbolzen 5 sind an dem einen Ende 2 und am Ende des Hubkörpers 5 je eine Lasche 43 für die Verbindung der Spannvorrichtung mit anderen Teilen vorgesehen.

Die Spannvorrichtung nach Fig. 4 entspricht im wesentlichen derjenigen nach Fig. 1 jedoch ist hier die Spindel 26 federnd mit dem Hubkörper 5 verbunden. Hierzu ist auf die Spindel 26 eine Führungshülse 44 aufgeschraubt, die einen Hals 45 aufweist, auf dem ein Satz Federn, z.B. Teilerfedern gelagert sind, die durch einen Sprengring 47 zusammengehalten werden. Die mittleren Teilerfedern sind in einer Nut 50 einer Bohrung 49 des Hubkörpers 5 gehalten. Die Relativbewegung des Hubkörpers 5 gegenüber der Führungshülse 44 erfolgt innerhalb eines Schlitzes 51, in den ein im Hubkörper 5 gelagerter Bolzen 52 ragt. Durch die beschriebene Federung wird ein brüsker Uebergung von der Belastung zur Entlastung und umgekehrt gedämpft.

In Fig. 5 ist eine Spannvorrichtung dargestellt, bei der anstelle des Elektromotors ein elektrischer Schrittmotor 53 mit einem Reduktionsgetriebe verwendet wird. Die Lageranordnung ist in gleicher Weise ausgeführt wie in Fig. 2, jedoch ist der Band 25 wegen des im Hinblick auf den verwendeten Schrittmotor 53 etwas grössere Durchmesser des Hohlprofils 1 ebenfalls grösser. Der Hubkörper 5 weist einen Führungskopf 55 auf, mit dem der Hubkörper im Innern des Hohlprofils 1 geführt ist, während der im Durchmesser kleinere Teil des Hubkörpers 5 im Führungsring 39 geführt ist. Am ende des Führungskopfes ist eine Verbindungsöse 56 angeordnet, während am einen Ende 2 des Hohlprofils 1 eine Lasche 43 als Verbindungsmittel vorgesehen ist.

Wesentlich ist noch, dass dem Schrittmotor 53 ein im Bereich des Führungskörpers 5 im Innern des Hohlprofils 1 angeordneter Weggeber 57 zugeordnet ist, mit welchem die Lage des Hubkörpers 5 geregelt wird. Der Weggeber 57 ist zweckmässig als digitaler Weggeber ausgebildet. In Fig. 5, 6 und 7 ist zudem die elektrische Zuleitung 58 mit einer Kupplung 59 zur Befestigung der Zuleitung 58 am Hohlprofil 1 dargestellt.

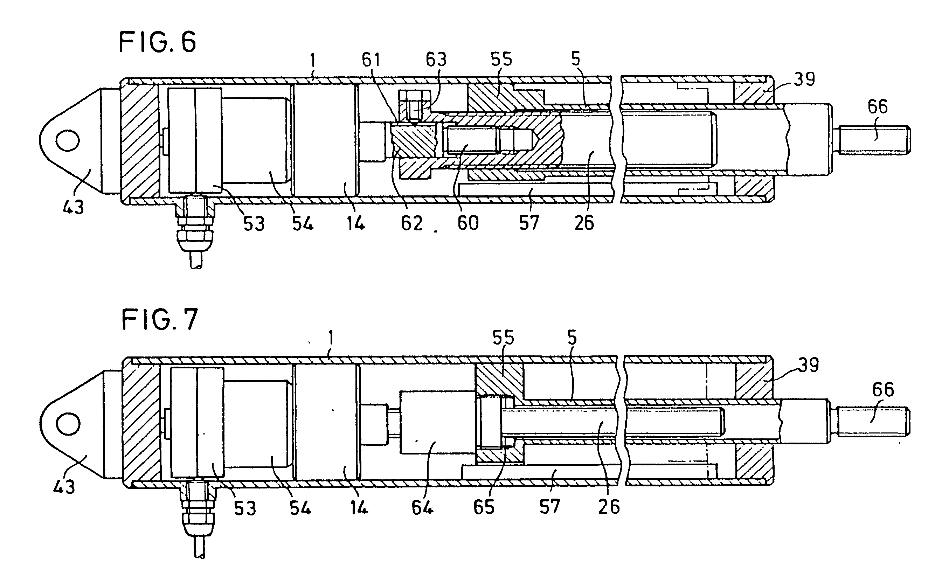

Bei den Spannvorrichtungen nach Fig. 6 und 7 sind ebenfalls Spannvorrichtungen mit einem Schrittmotor als elektrischen Antrieb vorgesehen. Bei der Ausführung nach Fig. 6 wird eine Doppelspindel verwendet, durch die dem Hubkörper zwei Geschwindigkeiten, eine kleinere und eine grössere erteilt werden können.

Die Doppelspindel weist die Spindel 26 auf, die mit dem Hubkörper 5 den dem Spindeltrieb in Fig. 1, 3, 4 und 5 entsprechenden Spindeltrieb bildet. In das antriebseitige Ende der Spindel 26 ist eine weitere Spindel 60 engeschraubt, die mit einem, eine Nut 61 aufweisenden Mitnehmerteil 62 verbunden ist, der seinerseits mit der Abtriebswelle des Reduktionsgetriebes 54 gekuppelt ist. In die Nut 61 ragt eine gefederte Raste 63, die am antriebseitigen Ende der Spindel 26 angeordnet ist. Bewegt sich der Hubkörper 5 ohne Belastung, ist die Kraftwirkung der Raste 63 ausrechend, so dass zwischen der Spindel 26 und der Spindel 60 keine Relativbewegung stattfindet. Entsteht eine Kraftwirkung auf den Hubkörper 5 schnappt die gefederte Raste 63 aus der Nute 61. Die dadurch entstehende Relativbewegung zwischen den Spindeln 26, 60 führt zu einer geringeren Geschwindigkeit des Hubkörpers 5. Die Spannvorrichtung nach Fig. 6 ist demnach in der Lage, mit zwei Geschwindigkeiten zu arbeiten, je nachdem kein oder eine geringe Belastung oder aber eine grosse Belastung auf den Hubkörper 5 wirkt.

Die Spannvorrichtung nach Fig. 7 weist einen Spindeltrieb mit einer Kugelgewindespindel auf. Ein Gehäuse 64, das die Kugeln und die notwendigen Kugelkanäle für den Umlauf der Kugeln enthält, ist in eine Gewindebohrung 65 des Führungskopfes 55 eingeschraubt, während die Spindel 26 wie bei den Spannvorrichtungen nach Fig. 1, 3, 4 und 5 mit der Austrittswelle des Reduktionsgetriebes 54 verbunden ist. Die übrigen Teile der Spannvorrichtung 7 entsprechen denjenigen nach Fig. 6. Bei beiden Spannvorrichtungen weist der Hubkörper 5 an seinem Ende einen Gewindebolzen 66 als Verbindungsmittel auf.

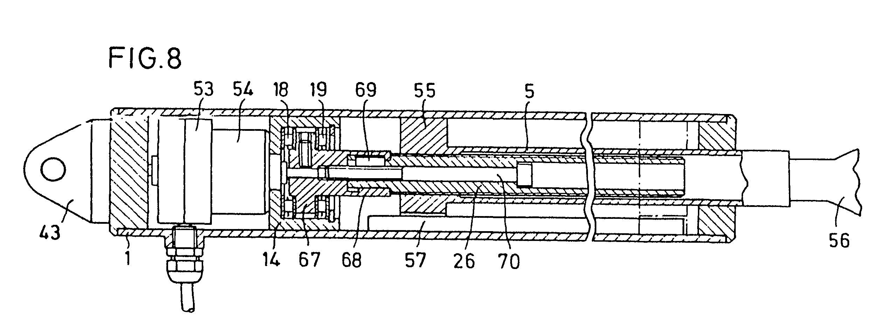

Die Spannvorrichtung nach Fig. 8 ist, wie z.B. die Spannvorrichtung nach Fig. 5, mit einem elektrischen Schrittmotor 53 mit einem Reduktionsgetriebe 54 versehen. An das Reduktionsgetriebe schliesst die Lageranordnung an, bestehend aus der Lagerbüchse 14, den Axialwälzlagern 18, 19 und einem Lagerkörper 67 mit einem Spindelstutzen 68. Die Spindel 26, in Fig. 8 als Hohlspindel ausgebildet, ist mittels einer Keilverbindung 69 im Spindelstutzen 68 drehfest gelagert und mit einem, im Lagerkörper 67 eingeschraubten Schraubenbolzen 70 gesichert. Die Spindelmutter der Spindel 26 ist der Führungskopf 55 mit dem daran befestigten Hubkörper 5, dessen freies Ende z.B. eine Verbindungsöse 56 trägt. Im Innern des Hohlprofils 1 ist wie bei der Ausführung nach Fig. 5, 6 und 7 ein Weggeber 57 eingebaut, mit dem eine Wegmessung durchgeführt werden kann.

Der Vorteil der Spannvorrichtung nach Fig. 8 besteht darin, dass der Spindelsatz, d.h. die Spindel 26 und der Führungskörper 55 mit dem Hubkörper 5 durch Lösen des Schraubenbolzens 70 vom Antrieb getrennt und z.B. durch eine andere Spindelgarnitur schnell und problemlos ersetzt werden kann.

Zusammengefasst zeichnen sich die beschriebenen Spannvorrichtungen durch einen einfachen und dadurch kostengünstigen Aufbau bei geringem Platzbedarf aus. Vorzugsweise kann der elektromotorische Antrieb mit Niederspannung betrieben werden, jedoch ist auch eine Ausführung für Netzspannung möglich. Der grundsätzliche Ausbau der Spannvorrichtung lässt trotzdem zahlreiche Varianten zur Einfacher Anbau von Endschaltern, einfacher Einbau einer Federung zwischen Hubkörper 5 und Spindel 26 sowie ein einfacher Uebergang auf Schrittmotoren, gegebenenfalls kombiniert mit Schrittgebern 57 zur Schaffung eines Regelkreises.

Die Lageranordnungen in Fig. 1, 2, 3, 5 und 8 weisen Wälzlager auf, jedoch können auch andere Lageranordnungen, z.B. mit Gleitlagern oder Gleit- und Wälzlagern, verwendet werden.

Die Anordnungen der beschriebenen Spannvorrichtungen im orthopädischen Bereich sind zahlreich; Kraftausübung auf Stützkorsett, Betätigung von künstlichen Gliedern und Bewegungshilfen. Die Anwendungen sind jedoch nicht auf das orthopädische Gebiet beschränkt. Vielmehr ist eine Anwendung überall dort denkbar, wo die besonderen Eigenschaften der beschriebenen Spannvorrichtungen zweckmässig eingesetzt werden können.

**Patentansprüche**

1. Elektrische Spannvorrichtung für eine orthopädische Justiervorrichtung, die ein mit einem elektromotorischen Antrieb (11, 12) und einem Spindeltrieb (5, 26) versehenes Hubwerk aufweist, dadurch gekennzeichnet, dass das Hubwerk in einem Hohlprofil (1) gelagert ist, das an dem einen Ende (2) mit Verbindungsmitteln (10) versehen ist und an dem andern, offenen Ende (4) eine Führung für einen in dem Hohlprofil (1) verschiebbar gelagerten Hubkörper (5) bildet, an dem Verbindungsmittel (10, 43, 56, 66) angeordnet sind, wobei die Spindel (26) des Hubwerkes zwischen dem elektromotorischen Antrieb (11, 12) und dem Hubkörper (5) mit einer Lageranordnung (14, 18, 19) zur Aufnahme entgegengesetzt wirkender Axialkräfte versehen ist.

2. Spannvorrichtung nach Anspruch 1, dadurch gekennzeichnet, dass der Hubkörper (5) am antriebseitigen Ende als Spindelmitter ausgebildet ist, die in dem Mantelrohr (1) geführt ist.

3. Spannvorrichtung nach Anspruch 1 oder 2, dadurch gekennzeichnet, dass das Hubwerk einen mit Niederspannung versehenen Elektromotor (11) mit einem Reduktionsgetriebe (12), vorzugsweise einem Planetengetriebe, aufweist, dessen Ausgangswelle (28) mit dem Spindeltrieb (5, 26) in Wirkungsverbindung steht.

4. Spannvorrichtung nach Anspruch 1, dadurch gekennzeichnet, dass die Lageranordnung Axiallager (18, 19) aufweist, die in einer Lagerbüchse (14) gelagert sind, die in dem Hohlprofil (1) eingesetzt und durch eine von aussen lösbare Sicherung (17) gehalten ist.

5. Spannvorrichtung nach Anspruch 4, dadurch gekennzeichnet, dass eine weitere lösbare Sicherung (30) zwischen der Getriebewelle (28) und der Spindel (26) vorgesehen ist, die nach Entfernen der Sicherung (17) einen Zugang (16, 34) zum Lösen der Spannen der weiteren Sicherung (30) aufweist.

6. Spannvorrichtung nach einem der Ansprüche 1 bis 5, dadurch gekennzeichnet, dass auf die Spindel (26) des Spindeltriebes (5, 26) eine Führungshülse (44) aufgeschraubt ist, auf der eine Federung (46), z.B. ein Tellerfedersatz, gelagert ist, die als nachgiebiges Verbindungsglied mit dem Hubkörper (5) gekuppelt ist.

7. Spannvorrichtung nach Anspruch 1, dadurch gekennzeichnet, dass der Elektromotor ein Schrittmotor (53) ist, an den ein Reduktionsgetriebe (54) angekuppelt ist, wobei die Abtriebswelle des Reduktionsgetriebes in Wirkungsverbindung mit dem Spindeltrieb (5, 26) steht und dem Hubkörper (5) ein im Innern des Hohlprofils (1) angeordneter Weggeber (57) zugeordnet ist.

8. Spannvorrichtung nach Anspruch 1, dadurch gekennzeichnet, dass in der Spindel (26) des Spindeltriebes (5, 26) antriebseitig eine weitere Spindel (60) schraubbar gelagert ist, die mit einem Mitnehmerteil (62) verbunden ist, in dessen Mitnehmernute (61) eine, im antriebseitigen Ende der Spindel (26) gelagerte, gefederte Raste (63) ragt, zwecks starrer Kupplung der beiden Spindeln (26, 60) bis zur Erreichung einer Grenzspindelbelastung bei einer ersten Verschubgeschwindigkeit und zwecks Einleitung einer Relativbewegung zwischen den Spindeln (26, 60) nach Anslösen der Raste (63) nach Ueberschreiten der Grenzspindelbelastung bei einer zweiten, gegenüber der ersten Vorschubgeschwindigkeit kleineren Vorschubgeschwindigkeit.

9. Spannvorrichtung nach Anspruch 1, dadurch gekennzeichnet, dass die Lageranordnung (14, 18, 19) einen Lagerkörper (67) mit einem Spindelstutzen (68) aufweist, wobei die als Hohlwelle ausgebildete Spindel (26) im Spindelstutzen (68) drehfest gelagert und am Lagerkörper durch einen Schraubenbolzen (70) gesichert ist.

**Revendications**

1. Dispositif de tension électrique pour un dispositif d'ajustage orthopédique, qui comporte un mécanisme de déplacement pourvu d'un entraînement électromotorisé (11, 12) et un mécanisme à broche (5, 26), caractérisé en ce que le mécanisme de déplacement est monté dans un profilé creux (1) qui est pourvu, à une extrémité (2), de moyens de liaison (10) et qui forme à l'autre extrémité (4) ouverte, un guidage pour un corps mobile (5) monté coulissant dans le profilé creux (1), corps mobile sur lequel sont disposés des moyens de liaison (10, 43, 56, 66), la broche (26) du mécanisme de déplacement étant pourvue, entre l'entraînement électromotorisé (11, 12) et le corps mobile (5, 26), d'un ensemble de paliers (14, 18, 19) destiné à absorber des forces axiales opposées.

2. Dispositif de tension selon la revendication 1, caractérisé en ce que le corps mobile (5) se présente, à l'extrémité côté entraînement, sous la forme d'un écrou de broche qui est guidé dans le tube d'enveloppe (1).

3. Dispositif de tension selon la revendication 1 ou 2, caractérisé en ce que le mécanisme de déplacement comporte un moteur électrique (11), alimenté en basse tension, avec un réducteur (12), de préférence un engrenage planétaire dont l'arbre de sortie (28) est en liaison active avec le mécanisme à broche (5, 26).

4. Dispositif de tension selon la revendication 1, caractérisé en ce que l'ensemble de palier comporte des paliers axiaux (18, 19) qui sont montés dans un coussinet (14), lequel est placé dans le profilé creux (1) et maintenu par une fixation (17) amovible de l'extérieur.

5. Dispositif de tension selon la revendication 4, caractérisé en ce qu'on prévoit une autre fixation (30) amovible, entre l'arbre d'entraînement (28) et la broche (26), qui, après enlèvement de la fixation (17), présente un accès (16, 34) pour desserrer l'autre fixation (30).

6. Dispositif de tension selon l'une quelconque des revendications 1 à 5, caractérisé en ce qu'on visse sur la broche (26) du mécanisme à broche (5, 26) une douille de guidage (44) sur laquelle sont montés des ressorts (46), par exemple un jeu de ressorts à disque, qui sont accouplés au corps mobile (5), pour former un organe de liaison élastique.

7. Dispositif de tension selon la revendication 1, caractérisé en ce que le moteur électrique est un moteur pas-à-pas (53) auquel est accouplé un réducteur (54), l'arbre de sortie du réducteur étant en liaison active avec le mécanisme à broche (5, 26) et un capteur de position (57), placé à l'intérieur du profilé creux (1), étant associé au corps mobile (5).

8. Dispositif de tension selon la revendication 1, caractérisé en ce qu'un visse dans la broche (26) du mécanisme à broche (5, 26), côté entraînement, une autre broche (60) qui est reliée à un autre élément entraîneur (62) dans la rainure (61) duquel s'engage un cliquet (63) monté, sous ressort, dans l'extrémité côté entraînement de la broche (26), en vue d'un accouplement rigide des deux broches (26, 60), jusqu'à obtention d'une charge de broche limite pour une première vitesse d'avance et en vue d'amorcer un déplacement relatif entre les broches (26, 60), après déclenchement du cliquet (63), lorsque la charge limite de broche est dépassée, pour une deuxième vitesse d'avance, inférieure à la première.

9. Dispositif de tension selon la revendication 1, caractérisé en ce que l'ensemble de paliers (14, 18, 19) comporte un corps de palier (67) avec une section de broche (68), la broche (26), se présentant sous la forme d'un arbre creux, étant montée fixe en rotation dans la section de broche (68) et fixée sur le corps de palier, par un boulon (70).

**Claims**

1. Electrical tensioning device for an orthopaedic adjustment device which has a stroke mechanism provided with an electric motor drive (11, 12) and a spindle drive (5, 26) characterised in that the stroke unit is mounted in a hollow section (1) which is provided at the one end (2) with connection means (10) and at the other open end (4) constitutes a guide (4), a stroke body (5) mounted slidably in the hollow section (1) on which connection means (10, 43, 56, 66) are arranged wherein the spindle (26) of the stroke unit is provided between the electric motor drive (11, 12) and the stroke body (5) with a bearing arrangement (14, 18, 19) for the receipt of oppositely directed axial forces.

2. Tensioning device according to Claim 1, characterised in that the stroke body (5) at the drivewards end is constructed as a spindle nut which is guided in the tubular casing (1).

3. Tensioning device according to Claim 1 or 2, characterized in that the stroke unit has an electric motor (11) supplied with low voltage with a reduction gear (12), preferably a planetary gear, the output shaft (28) of which is in operative connection with the spindle drive (5, 26).

4. Tensioning device according to Claim 1, characterized in that the bearing arrangement has axial bearings (18, 19) which are mounted in a bearing shell (14) which is set in the hollow section (1) and is held by an externally releasable securement (17).

5. Tensioning device according to Claim 4, characterised in that a further releasable securement (30) is provided between the drive shaft (28) and spindle (26) which, after removal of the securement (17), has an access (16, 34) for releasing the tensioning of the further securement (30).

6. Tensioning device according to one of Claims 1 to 5, characterised in that on the spindle (26) of the spindle drive (5, 26) there is screwed a guide sleeve (44) on which as springing (46), e.g. a set of plate springs, is mounted which is coupled as a resilient coupling member wiht the stroke body (5).

7. Tensioning device according to Claim 1, characterised in that the electric motor is a stepping motor (53) to which a reduction gear (54) is coupled wherein the output shaft to the reduction gear is in operative connection with the spindle drive (5, 26) and there is arranged relative to the stroke body (5) a path sensor (57) arranged in the interior of the hollow section (1).

8. Tensioning device according to Claim 1, characterised in that in the spindle (26) of the spindle drive (5, 26) on the drive side there is threadedly mounted a further spindle (60) which is connected with an entrainer part (62) in the entrainer groove (61) of which a spring catch (63) mounted in the drivewards end of the spindle (26) projects with the object of rigid coupling of both spindles (26, 60) up to the achievement of a limiting spindle loading at the first forward feed speed and with the object of introducing a relative movement between the spindles (26, 60) after release of the catch (63) on exceeding the limiting spindle load at a second forward feed speed smaller relative to the first forward feed speed.

9. Tensioning device according to Claim 1, characterised in that the bearing arrangement (14, 18, 19) has a bearing body (67) with a spindle post (68) wherein the spindle (26) constituted as a hollow shaft is mounted rotatably fixed in the spindle post (68) and is secured to the bearing body by a threaded pin (70).

FIG. 1

FIG. 2

EP 0 160 274 B1

FIG.3

FIG.4

FIG.5

EP 0 160 274 B1

FIG.6

FIG.7

3

FIG.8

4